Europäisches Patentamt

European Patent Office

Office européen des brevets

(11) Publication number: **0 175 841 B1**

# EUROPEAN PATENT SPECIFICATION

(45) Date of publication of patent specification: 06.03.91

(51) Int. Cl.⁵: **A61K 39/10**, A61K 39/05, A61K 39/08

(21) Application number: 85106303.2

(22) Date of filing: 22.05.85

(54) Method for the production of pertussis component vaccine and combined vaccine of pertussis antigen; diphtheria toxoid and tetanus toxoid.

(30) Priority: 22.09.84 JP 198899/84

(43) Date of publication of application:
02.04.86 Bulletin 86/14

(45) Publication of the grant of the patent:
06.03.91 Bulletin 91/10

(84) Designated Contracting States:
AT BE CH DE FR GB IT LI LU NL SE

(56) References cited:
EP-A- 0 003 916      EP-A- 0 077 646
EP-A- 0 118 885      EP-A- 0 140 386
EP-A- 0 159 003      EP-A- 0 170 162

INFECTION AND IMMUNITY, vol. 41, no. 1, July 1983, pages 313-320, American Society for Microbiology; Y. SATO et al.: "Separation and purification of the hemagglutinins from Bordetella pertussis"

(73) Proprietor: **Juridical Foundation The Chemo-Sero-Therapeutic Research Institute**
**668, Okubo Shimizu-machi**
**Kumamoto-shi Kumamoto-ken(JP)**

(72) Inventor: **Ginnaga, Akihiro**
**9-12, Hakenomiya 3-chome**
**Kumamoto-shi Kumamoto-ken(JP)**
Inventor: **Sakoh, Mitsuo**
**119-5, Kaminogo-machi**
**Kumamoto-shi Kumamoto-ken(JP)**
Inventor: **Kitagawa, Hisashi**
**4-66, Hieda-machi**
**Kumamoto-shi Kumamoto-ken(JP)**
Inventor: **Sakuma, Shin**
**2056-32, Nagamine-machi**
**Kumamoto-shi Kumamoto-ken(JP)**
Inventor: **Koba, Hiroshi**
**23-2, Kurokami 2-chome**
**Kumamoto-shi Kumamoto-ken(JP)**

Inventor: **Nishihara,Tsukasa c/o Juridical Foundation The Chemo-Sero-Therapeutic-Research Inst. 668, Okubo Shimizu-machi Kumamoto-shi Kumamoto-ken(JP)**
Inventor: **Hirashima, Sadahiro 7-4, Minami Funatsu-machi 3-chome Omuta-shi Fukuoka-ken(JP)**

(74) Representative: **Vossius & Partner Siebertstrasse 4 P.O. Box 86 07 67 W-8000 München 86(DE)**

## Description

The present invention relates to a method for the production of a pertussis component vaccine and a combined vaccine of pertussis antigen, diphtheria toxoid and tetanus toxoid. More particularly, it relates to an improved method for the production of a pertussis component vaccine by mixing a highly purified F-HA (Filamentous Hemagglutinin) or a toxoid thereof which is obtained from a culture of Bordetella pertussis by a specific method as described hereinafter and a highly purified toxoid of LPF-HA (Leucocycosis-Promoting Factor Hemagglutinin) which is obtained from a culture of B. pertussis by a known method or a specific method as described hereinafter, and to a combined vaccine of pertussis antigen, diphtheria toxoid and tetanus toxoid wherein the above improved pertussis component vaccine is used as the pertussis antigen component.

Pertussis is one of the infectious diseases and appears frequently in babies and children and hence is very important disease in view of public health. Particularly, babies suffered from this disease occasionally show very heavy symptom and sometimes result in death. It is well known that this disease can be protected by a previous vaccination, and for such a purpose, there is widely used an inactivated vaccine prepared from whole cells of B. pertussis phase I. However, the inactivated vaccine containing whole cells has significant side reaction, and hence, inoculation of vaccine had been prohibited temporarily in the past. The disease in babies and children caused by B. pertussis is still one of the most important social problems, and hence, it has been required to develop a pertussis vaccine with no side reaction.

Sato et al. had succeeded in the production of precipitated-purified pertussis vaccine which is an epochal component vaccine and had been found based on basic study on protective antigens (cf. Japanese Patent Publication No. 5203/1982). This vaccine comprises mainly as the protective antigens HA fraction containing F-HA and LPF-HA and shows excellent protective effect with little side reaction such as fever, and hence, it has already practically been used.

This precipitated pertussis vaccine is prepared by inoculating B. pertussis phase I in an appropriate medium, subjecting it to a static culture at about 35 ° C for 5 days, centrifuging the culture broth, separating the supernatant fluid, adding ammonium sulfate to the supernatant fluid so as to become about 50 % saturation or adding alcohol thereto, separating the resulting precipitates by centrifuging at 10,000 r.p.m. for 30 minutes, extracting the precipitates with a buffer supplemented with sodium chloride, subjecting the extract to a sucrose density gradient centrifugation in a usual manner to collect the pertussis HA fraction and remove endotoxin fraction, and then treating the resulting HA fraction with formalin to make detoxification. This vaccine is called as a component vaccine [cf. Sato Y., et al.; The Lancet (1), 122-126 (1984)]. However, as is seen in this literature, it contains F-HA and LPF-HA as the main pertussis vaccine component as well as other components. Moreover, it is recognized that the amount of such components can not freely be controlled, and hence it should rather be called as "acellular vaccine".

The vaccine prepared by the Sato et al. is further mixed with diphtheria toxoid and tetanus toxoid, and optionally treated with an aluminum adjuvant and further mixed with a stabilizer such as gelatin, glucose, etc. to obtain precipitated-purified pertussis antigens, diphtheria toxoid and tetanus toxoid. Recently, however, there have been issued various clinical reports of topical reactions such as redness, swelling, induration, etc., and hence the vaccine is not necessarily complete.

Furthermore, there has been noticed the role of F-HA and LPF-HA which are produced by B. pertussis phase I strain as an infection-protecting antigen, and the important role has been reported in various literatures [cf. Sato, Y. et al.; Infect. Immun., 31 , 1223 - 1231 (1981); Sato, Y. et al.; Seminars in Infectious Diseases IV, Bacterial Vaccine, 380 - 385 (1982)]. In order to use the F-HA and LPF-HA as the component of pertussis vaccine without undesirable side effect such as local reactions, it is important to highly purify them, and further, it is also important to be able to be produced simply and in a large quantity for the purpose of producing the antigen in an amount sufficient to use as a vaccine.

It is known that F-HA can be isolated and purified by subjecting the supernatant of B. pertussis culture to fractionation with ammonium sulfate, subjecting the resultant to a sucrose density gradient centrifugation, followed by gel filtration twice [cf. Sato, Y. et al.; Infect. Immun., 9 , 801 (1974)]. However, this method requires a plenty of steps and hence is very complicated, and further gives the desired F-HA only in a low yield, and hence, this method can not be used in an industrial scale.

It is also known to purify B. pertussis F-HA by an ion exchange chromatography and a gel filtration [cf. Arai, H. et al.; Infect. Immun., 25 , 460 (1979)]. However, according to this method, the desired F-HA is obtained only in a low yield, and further, it is very difficult to remove undesirable B. pertussis endotoxin, and hence, this method can not practically be used, either.

Other known methods are a combination of hydroxyapatite adsorption chromatography, haptoglobin affinity chromatography, ammonium sulfate fractionation, and gel filtration [cf. Cowell, J. L. et al.; Seminars

3

in Infectious Diseases IV, Bacterial Vaccine, 37 , 1 (1982)]. However, this method requires a plenty of steps and hence is complicated, and further, the F-HA is obtained only in a low yield. Besides, the used hydroxyapatite is very expensive. From these many drawbacks, the above methods are not suitable for producing F-HA in an industrial scale.

There have also been known some methods for the separation and purification of LPF-HA, for instance, a method comprising salting out a culture medium of B. pertussis with ammonium sulfate, extracting and dialyzing, and then subjecting the thus obtained material to ion exchange chromatography, gel filtration [cf. Arai, II. et al.; Biochimica et Biophysica Acta, 444 , 765 (1976)] or a method by sucrose concentration gradient centrifugacion [cf. Sato, Y. et al.; Infect. Immun., 6 , 897 - 704 (1972)]. According to such known methods, however, it is very hard to obtain the desired LPF-HA which shows single band in the purification analysis by electrophoresis, and its yield is very low.

In order to obtain the desired highly pure LPF-HA in a comparatively large amount, it is also proposed that a supernatant of culture media of B. pertussis is passed through a column packed with hydroxyapatite to adsorb LPF-HA thereon, followed by washing, eluting and then subjecting to affinity chromatography with concanavalin A-Sepharose (Con A-Sepharose, manufactured by Pharmacia) [cf. Yajima, M. et al.; J. Biochem., 83 , 295 - 303 (1978)]. However, the affinity chromatography using concanavalin A as a ligand not only has an affinity with LPF-HA but also can adsorb saccharides, glycolipids and also other glycoproteins, and hence, it adsorbs other pertussis cell components such as cell membrane components, which results in difficulty of isolation of the desired highly pure LPF-HA. Moreover, the treatment with a column of hydroxyapatite takes a long time, which may result in lowering of LPF-HA activity, and further, because of expense of hydroxyapatite, the desired LPF-HA is hardly obtainable in a low cost in an industrial scale. Thus, it is not suitable as an affinity chromatography for LPF-HA.

Since it has recently been found that human haptoglobin binds specifically to LPF-HA, it has been tried to purify LPF-HA by an affinity chromatography using as a ligand the human haptoglobin instead of the above concanavalin [cf. Irons, L. et al.; Biochimica et Biophisica Acta, 580 , 175 - 185 (1979), and Cowell, J. et al.; Seminars in Infectious Diseases IV, Bacterial Vaccine, 371 - 379 (1982)]. There has also been proposed a method for collecting LPF-HA which comprises fracturing mechanically B. pertussis cells, extracting LPF-HA from the cell components, subjecting it to ammonium sulfate fractionation, and then, subjecting the thus obtained material to affinity chromatography using as a ligand plasma sialoproteins such as haptoglobin or ceruloplasmin, or sialoproteins such as salivary mucin (cf. British Patent First Publication 2,015,531) . These methods by affinity chromatography using as a ligand haptoglobin, etc. are expected as an industrial method for producing a purified LPF-HA.

Thus, there is never known any method for the production of F-HA having satisfactory purity in an industrial scale, and as to LPF-HA, only the above method by affinity chromatography using as a ligand haptoglobin is known as an industrially acceptable method.

An object of the present invention is to provide an improved industrial method for producing a pertussis component vaccine which does not show undesirable side effect by using highly purified F-HA and LPF-HA. Another object of the invention is to provide a method for producing a combined vaccine of pertussis antigen, diphtheria toxoid and tetanus toxoid by using the pertussis component vaccine.

The pertussis component vaccine

of the present invention is produced by mixing a highly purified F-HA which is obtained by the steps of treating a culture of B. pertussis with a cellulose sulfate gel, a polysaccharide gel chemically bound with dextran sulfate, or a crosslinked polysaccharide sulfate gel, thereby adsorbing F-HA on the gel, and then eluting F-HA from the gel, with a highly purified LPF-HA which is obtained by affinity chromatography using as a ligand haptoglobin as disclosed in GB-A-2,015,531 or by an improved method found by some of the present inventors as described hereinafter.

The combined vaccine of the present invention can be prepared by mixing the above pertussis component vaccine with diphtheria toxoid and tetanus toxoid.

The desired highly purified F-HA is prepared by the following methods, which are described in Japanese Patent Application Nos. 75314/1984, 84778/1984 and 91631/1984.

That is, B. pertussis is cultured in a conventional medium, such as Cohen-Wheeler medium or Stainer-Scholte medium, in a usual manner, such as stationary culture, shake culture, or spinner culture (this is synonym of shaking culture, aeration culture, and aeration spinner culture). The culture broth thus obtained is subjected to centrifugation to remove the cells and the supernatant is separated out. Alternatively, the cells are fractured and thereafter the mixture is subjected to centrifugation and the supernatant is separated out. The supernatant is subjected to the specific purification by gel adsorption chromatography as it stands or after partially purified by a conventional method. The previous purification such as salting out, extraction, ultracentrifugation, etc. is not necessarily applied, but the supernatant can be directly subjected to

4

chromatography with cellulose sulfate gel, a polysaccharide gel chemically bound with dextrane sulfate, or a crosslinked polysaccharide sulfate gel, and hence, the purification can be done in a simple step.

The sulfuric acid ester of cellulose used as the cellulose sulfate gel in the present invention is obtained by sulfating a cellulose, preferably a crystalline cellulose or cellulose having crystalline area and non-crystalline area. The sulfuric acid ester or cellulose thus obtained retains well the original shape (preferably spherical shape) of the starting material and is insoluble in an aqueous medium and has excellent physical stability, and hence, is suitable as a gel of chromatography. These starting celluloses are commercially available, for example, Abicel (manufactured by Asahi Kasei, Japan), Cellulofine GC-15, GH-25, GC-100, or GC-200 (manufactured by Chisso Corp., Japan). The sulfation of the cellulose can be carried out by a conventional method, for example, by treating a gel of cellulose with chlorosulfonic acid, anhydrous sulfuric acid, or other sulfating agent in an organic solvent (e.g. pyridine).

The polysaccharide gel chemically bound with dextran sulfate is produced by chemically binding a dextran sulfate to a polysaccharide gel derivative. Various products of the dextran sulfate are commercially available, among which the products used usually for biological purposes are preferably used. The polysaccharide gel derivative includes gel derivatives which are prepared by subjecting a polysaccharide (e.g. agarose, dextran, cellulose, etc.) to conventional treatments for giving properties suitable for using as a carrier for chromatography, such as crystallization purification treatment, three-dimensional crosslinking, molding, etc. These products are also commercially available and include, for example, an agarose gel such as Sepharose (manufactured by Pharmacia, Sweden), a dextran gel such as Sephadex (manufactured by Pharmacia), a cellulose gel such as Abicel (manufactured by Asahi kasei, Japan). The chemical binding of the dextran sulfate and the polysaccharide can be done by various methods, for example, by a method of Anderson et al. using cyanobromide (cf. Japanese Patent First Publication No. 114018/1977), or a method using cyanobromide and also lysine (as a spacer) [cf. Bryan M. Turner et a-l.; Biochimica et Biophysica Acta, 659 , 7-14 (1981)]. One product of dextran sulfate - agarose gel is already on the market, for example, Dextrane sulfate-Sepharose CL 4B (manufactured by Pharmacia).

The sulfuric acid ester of a crosslinked polysaccharide includes a sulfuric acid ester of polysaccharides, such as dextran, celluloses, agarose, which is crosslinked with a crosslinking agent, such as epichlorohydrin, dichlorohydrin, dibromohydrin, ethylene glycol bisepoxypropyl ether. The crosslinked polysaccharides are commercially available, for example, crosslinked dextrans such as Sephadex G-10, G-25, G-50, and G-100 (manufactured by Pharmacia, Sweden), crosslinked agaroses such as Sepharose CL-2B, CL-4B, and CL-6B (manufactured by Pharmacia), and crosslinked celluloses such as Cellulofine GCL-25, GCL-90 (manufactured by Chisso Corp., Japan). The sulfation of the crosslinked polysaccharide can be carried out by a conventional method, for example, by treating a gel of the crosslinked polysaccharide with chlorosulfonic acid, anhydrous sulfuric acid, or other sulfating agent in an organic solvent (e.g. pyridine).

The isolation and purification of F-HA from a culture of B. pertussis with these gels are carried out in the following manner.

The cellulose sulfate gel, dextran sulfate-polysaccharide gel and crosslinked polysaccharide sulfate gel are previously equilibrated with an appropriate buffer having a neutral pH (e.g. pH 6 - 9), a specific conductivity of about 5 to 25 mS/cm, such as 0.2 M sodium chloride-added 0.01M phosphase buffer, and then used for adsorption of F-HA (mS/cm = Millisiemens pro cm).

The purification treatments such as adsorption of F-HA onto the cellulose sulfate gel or other gels, washing of the gels adsorbing the F-HA and elution of the F-HA can be carried out by a conventional industrially employed operation such as batch method or column method. In case of batch method, the cellulose sulfate gel or other gels are added to a culture of B. pertussis, and the mixture is gently stirred at pH 6.0 - 9.0, at a temperature of 0° - 30°C for 10 to 60 minutes, whereby F-HA is adsorbed onto the gels. The culture of B. pertussis used in the above method is usually regulated to a specific conductivity in the range of 5.0 to 25.0 mS/cm by concentrating or diluting it. A supernatant of the culture solution may also be used as it stands, when it has the specific conductivity range as mentioned above.

After completion of adsorption, the culture - gel mixture is added onto a filter, and the gel is separated from the filtrate by suction. The separated gel is washed by suction with an appropriate buffer having a specific conductivity of about 5 to 25 mS/cm and a pH of about 5.0 to 10.0, for example, 0.2 M sodium chloride-added 0.02 M McIlvaine's buffer, 0.2 M sodium chloride-added 0.01 M phosphate buffer, or 0.2 M sodium chloride-added 0.01 M Tris-HCl buffer, etc. Thereafter, the adsorbed F-HA is eluted with an appropriate buffer having a pH of about 5.0 to 10.0 and a specific conductivity of about 25 to 130 mS/cm (larger specific conductivity than that of the above buffer for washing), such as, for example, 1.5 M sodium chloride-added McIlvaine's buffer, 1.5 M sodium chloride-added phosphate buffer, etc.

In case of column method, the starting solution to be treated, the buffer for washing and the buffer for elution are the same as those used in the batch method as mentioned above. The speed of passing the solution is controlled in the range of about 10 ml/cm²/hour to 500 ml/cm²/hour.

According to the above purification method, the F-HA in the culture of B. pertussis is specifically remarkably adsorbed, and hence, the purification degree of F-HA becomes several ten folds and further the recovery rate of F-HA reaches to more than 75 %, in some conditions, to from more than 90 % to almost 100 %. Beside, the purified F-HA has so high specific activity as about 3.7 - 11 x 10⁴ HA unit/mg protein, usually 4 - 8 x 10⁴ HA unit/mg protein [measured by a chicken blood cell agglutination test, of. Sato, Y. et al.; Infect. Immun., 7 , 929-999 (1973)], and when it is shown in ELISA activity, it has 0.9 - 1.3 x 10⁵ F-HA-Ab-ELISA unit/mg protein [measured by F-HA.ELISA method, cf. Sato, Y. et al.; Infect. Immun., 41 , 313-320 (1983)], and further, forms a single band in a polyacrylamide disc electrophoresis analysis (pH 4.5), and B. pertussis endotoxin is almost completely removed.

According to the above purification method, the desired F-HA can be isolated from the starting culture of B. pertussis in a high yield and high purity with very simple operation. Besides, the chromatography adsorbent can be prepared in a low cost and also can be used repeatedly without deterioration. Accordingly, the method is excellent from economical viewpoint. Thus, the above purification method is very excellent as an industrial method for production of a highly purified F-HA. If necessary, the purification may be combined with conventional purification methods, such as sucrose density gradient ultracentrifugation, ion exchange chromatography, etc.

The purified F-HA thus obtained is very pure and hence can be used as a starting material for producing the final vaccine as it stands, but may optionally be used after being converted into a toxoid by a conventional method using formalin.

The purified LPF-HA may be obtained by the method as disclosed in GB-A-2,015,531 but is preferably prepared by the following purification method.

One method is as follows (cf. Japanese Patent Application No. 206598/1983, U.S. Serial No. 666,172).

The starting culture medium obtained by culturing B. pertussis in a usual manner is subjected to an affinity chromatography using a denatured ceruloplasmin as a ligand, whereby the endotoxins of B. pertussis are removed off during the purification of LPF-HA to give a highly purified LPF-HA in a high yield.

The denatured ceruloplasmin used in the purification is obtained by denaturing ceruloplasmin of animal origin by various methods, for example, by heating ceruloplasmin at 60 to 85°C for 1 to 24 hours, or by treating ceruloplasmin with a denaturing agent, such as a sulfide (e.g. sodium sulfide, ammonium sulfide, etc.), L-ascorbic acid, a reducing sugar (e.g. D-glucose, D-galactose, D-mannose, D-fructose, maltose, lactose, etc.), a reducing agent (e.g. acetaldehyde, formic acid, oxalic acid, mercaptoethanol, diethyldithiocarbamate, etc.), a cyano compound (e.g. sodium cyanide, sodium thiocyanate, potassium thiocyanate, etc.), a chelating agent (e.g. EDTA, nitrotriacetic acid (NTA), triethylenetetramine-hexaacetic acid (TTHA), etc.), whereby the copper ion (Cu⁺⁺) contained in ceruloplasmin is reduced or a part or whole of the copper ion is isolated and removed.

The above denaturing means may be applied to alone or in combination of two or more, and the denaturing may be applied to after immobilizing ceruloplasmin (i.e, a ligand) into a matrix (i.e. a suppoting carrier). The denaturing can easily be carried out by dialyzing one volume of a 0.1 to 0.5 w/v % solution of ceruloplasmin in physiological saline solution against 10 to 200 volume of the following buffer. That is, in case of using sodium sulfide, ammonium sulfide, or sodium cyanide as the denaturing agent, a 0.01 to 0.1 M phosphate buffer containing 0.01 to 1.0 M of the denaturing agent (pH 6.0 - 8.0) is used, and the dialysis is carried out at 0 to 60°C for 1 to 10 hours. In case of using L-ascorbic acid or ruducing sugars as the denaturing agent, a 0.01 to 0.1 M acetate buffer containing 0.01 to 1.0 M of the denaturing agent (pH 4,0 - 6.0) is used, and the dialysis is carried out at 0 to 15°C for 24 to 36 hours. In case of using acetaldehyde, formic acid, oxalic acid, mercaptoethanol, diethyldithiocarbamate, or the like as the denaturing agent, a 0.01 to 0.1 M phosphate buffer containing 0.01 to 0.1 M of the denaturing agent (pH 6.0 - 8.0 ) is used, and the dialysis is carried out at 0 to 30°C for 0.5 to 3 hours. In case of using thiocyanates as the denaturing agent, 0.01 to 0.1 M phosphate buffer containing 0.1 to 3.0 M of the denaturing agent (pH 6.0 - 8.0) is used, and the dialysis is carried out at 0 to 30°C for 0.5 to 5.0 hours. In case of using EDTA, NTA, TTHA as the denaturing agent, a 0.01 to 0.1 M phosphate buffer containing 0.01 to 1.0 M of the denaturing agent (pH 6.0 - 8.0) is used, and the dialysis is carried out at 0 to 30°C for 0.5 to 5.0 hours.

The starting ceruloplasmin is commercially available, or may be obtained by subjecting blood plasma to alcohol fractionation by Cohn method as Fraction IV, or may be obtained by separating from human or other animals blood and then purifying.

The affinity chromatography of culture media of B. pertussis with the above denatured ceruloplasmin is usually carried out in the following manner.

An affinity gel is prepared by the method of Axe'n et al. [cf. Axe'n et al.; Nature, 214 , 1302 - 1304 (1967)], i.e. by immobilizing the denatured ceruloplasmin into a matrix of cepharose, agarose, cellulose, or dextran, etc. which are activated with cyano bromide. The affinity gel is contacted with the culture media of B. pertussis in column method or batch system, whereby LPF-HA contained in the media is adsorbed onto the gel, followed by washing the gel with an appropriate buffer to remove contaminants and then eluting LPF-HA with an eluent.

The starting culture media of B. pertussis include the same as used for the purification of F-HA as mentioned hereinbefore, for example, culture media obtained by culturing B. pertussis phase I strain in a conventional liquid medium, such as Cohen-Wheeler medium or Stainer-Scholte medium, in a usual manner, such as stationary culture, shake culture, or spinner culture. Preferably, the culture media are subjected to centrifugation or filtration in order to remove cells. Besides, the solution passed through in the purification of F-HA as disclosed hereinbefore may also be used as the starting material. According to this purification method of LPF-HA, the culture can be applied to the affinity chromatography as they stand, i.e. without subjecting to various pre-treatments such as salting out, extraction, dialysis, ultracentrifugation, concentration, equilibration, or the like, and hence, the procedure is very simple.

According to a column method, the affinity gel is packed into a column, and the starting culture medium of B. pertussis is passed through the column at a flow rate of 10 ml/cm$^2$/hour to 500 ml/cm$^2$/hour.

According to a batch method, the culture medium of B. pertussis is entered into a vessel, and thereto is directly added the affinity gel, and the mixture is stirred for about 30 minutes to about 3 hours, preferably for about one hour.

The amount of the affinity gel is not critical, but usually, 0.1 ml of affinity gel is used for adsorbing 10,000 to 20,000 $\mu$g of LPF-HA (in protein amount).

Washing of the LPF-HA-adsorbed affinity gel is usually carried out with a buffer having a pH 4.0 - 9.0 and a specific conductivity of 10 to 150 mS/cm. For example, by using a 0.01 to 0.1 M phosphate buffer (pH 6.0 - 8.0) containing 0.1 to 1.0 M sodium chloride, the washing is carried out by flowing the buffer in a volume of several tens to 100 times as much as the volume of the column in case of the column method, or by treating with the buffer in a volume of several to several hundreds times as much as the volume of gel in the batch method. By the washing, endotoxin of B. pertussis contained in the starting material is effectively removed. This is also one of the characteristics of the purification method, which is superior to the conventional purification methods.

After the above washing step, the LPF-HA adsorbed onto the ligand is eluted in a usual manner by using conventional eluents, such as chaotropic salts (e.g. salts which can release chaotropic ions such as I$^-$, ClO$_4^-$, CF$_3$COO$^-$, SCN$^-$, CCl$_3$COO$^-$, etc.), ethylene glycol, dioxane, urea, guanidine hydrochloride, EDTA, or the like.

According to the affinity chromatograpy of this method, the desired product can be obtained in a high yield such as more than 90 % in case of the starting material having a pH 4.0 to 10.0.

The LPF-HA obtained by the above method has a high purity as more than 90 %, occasionally more than 95 % (in the analysis by electrophoresis, pH 4.5). Besides, the product has a specific activity of 0.9 - 1.6 x 10$^5$ LPF-Hp-ELISA unit/mg of protein, usually 1.1 - 1.2 x 10$^5$ LPF-Hp-ELISA unit/mg of protein [measured by ELISA analysis, cf. Sato et al.; Symposium on Toxins, Proceeding of the 28th Symposium on Toxins, 141-144 (1981)], which can not be achieved by the conventional purification methods. Superiority of the product is also clear from the results in biological activities tests of the LPF-HA product, such as Limurus test and pyrogen test in rabbit. That is, in case of this purification method, the undesirable endotoxin is almost completely removed. Besides, the above method has an advantage that there can be avoided the problems such as hepatitis virus and other infectious factors which are observed in case of using human blood plasma components.

For instance, in case of using a ceruloplasmin denatured by heat treatment, the specific adsorbability of LPF-HA and capacity of the adsorption are significantly enhanced, and further, the hepatitis virus or the like is effectively removed. Moreover, in case of using as the ligand a ceruloplasmin denatured by 1-ascorbic acid, sodium cyanide etc., even if the denatured ceruloplasmin is heat-treated at 60 °C for 10 - 15 hours, the gel can show sufficient purification capacity as like as the product not subjected to the heat treatment. Besides, even when a ceruloplasmin subjected to heat treatment at 60 °C for 10 hours is further treated with a denaturing agent (e.g. 1-ascorbic acid or sodium cyanide), the denatured product can show the same or more properties as the product denatured with the denaturing agent without subjecting to the heat treatment. Thus, the above method is also excellent in view of no danger of contamination of hepatitis virus or the like and further sufficient removal of endotoxin of B. pertussis.

The affinity chromatography wherein the denatured ceruloplasmin as obtained above is used as the ligand can give the desired LPF-HA in a high yield and high purity from the starting culture medium of B. pertussis by a simple procedure. Besides, the affinity gel can repeatedly be used for several tens to several hundreds of times or more, which is advantageous in view of low cost. The method of the present invention is also advantageous in that endotoxin of B. pertussis can almost be removed. Accordingly, the above first purification method is very useful as an industrial method for obtaining a highly pure LPF-HA. Besides, the LPF-HA thus obtained is highly pure and does not contain other proteins, lipids and saccharides as well as endotoxin, and hence, is useful for the preparation of the desired pertussis vaccine.

The second method for preparing LPF-HA in a high purity is done by using gels such as a cellulose sulfate gel, a polysaccharide gel chemically bound with dextrane sulfate, or a crosslinked polysaccharide sulfate gel (cf. Japanese Patent Application Nos. 150,945/1984, 175710/1984 and 190744/1984) which are the same as used for the purification of F-HA as discribed herinbefore. The method comprises the steps of treating a culture of B. pertussis with the gels, thereby adsorbing LPF-HA on the gels, followed by eluting LPA-HA.

The starting culture of B. pertussis used in the second method is the same as that used in the purification method of F-HA or that used in the first purification method of LPF-HA as described hereinbefore.

The purification method of LPF-HA using the cellulose sulfate gel and other gels is carried out as follows.

The starting LPF-HA-containing solution may be prepared by centrifuging a culture of B. pertussis, diluting the supernatant with distilled water or a buffer so as to become a specific conductivity of 0.1 to 5.0 ms/cm, and then subjecting to the adsorption treatment. However, since the supernatant contains usually F-HA which has also affinity to the cellulose sulfate gel and other gels, the supernatant may be subjected to chromatography with cellulose sulfate gel or other gels under the conditions that LPF-HA is not adsorbed but F-HA is adsorbed (wherein the starting solution regulated to a specific conductivity of 5.0 - 25.0 mS/cm and pH 5 - 9 is passed through a column packed with a cellulose sulfate gel or other gels, which is equilibrated with a buffer of specific conductivity of 5.0 - 25.0 mS/cm and pH 5 - 9), and then the fraction passed through the column which does not contain F-HA and contains a large amount of LPF-HA is subjected to the adsorption treatment.

The purification treatment comprising adsorption of LPF-HA onto the cellulose sulfate gel or other gels, washing of the gels adsorbing the LPF-HA and elution of the LPF-HA can be carried out by a conventional industrially employed operation such as batch method or column method. In case of column method, the cellulose sulfate gel or other gels are packed in a column, and it is previously equlibrated by passing through an appropriate buffer having a specific conductivity of 0.1 to 5.0 mS/cm and a pH of 5.0 to 9.0, for example a 0.02 M McIlvaine's buffer (pH 5.2), and then it is used for the adsorption of LPF-HA.

In the adsorption, the LPF-HA-containing solution is usually regulated to a pH of 5.0 to 9.0 and a specific conductivity of 0.5 to 5.0 m$^S$/cm, and then passed through the column packed with the cellulose sulfate gel or other gel to adsorb LPF-HA. Thereafter, the column is washed with the same buffer as used for the above equilibration, by which contaminated materials are washed out.

The elution of LPF-HA is usually carried out by passing through an appropriate buffer having a pH of 5.0 to 9.0 and a specific conductivity of 5.0 m$^S$/cm or more, preferably by stepwise elution or salt concentration gradient elution. That is, when a diluted supernatant obtained by centrifugation of a culture of B. pertussis is used as the starting material, F-HA is also adsorbed together with LPF-HA in the above adsorption step, and hence, it is necessary to elute LPF-HA under the condition that can elute LPF-HA but not F-HA. It is as follows. Firstly, an appropriate buffer having a pH of 5 to 9 and a spcific conductivity of 5 to 100 mS/cm, preferably 50 to 60 m$^S$/cm (for example, a 0.7 M sodium chloride-added 0.02 M McIlvaine's buffer) is passed through the column, by which a fraction containing LPF-HA is recovered. Thereafter, a buffer having a specific conductivity of larger than that of the above buffer for elution (e.g. a specific conductivity of 100 to 300 mS/cm) is passed through, by which F-HA and other impurities are eluted out, followed by equilibrating the cellulose sulfate gel or other gel in order to re-use the gel.

The most preferable elution is carried out by a salt concentration gradient elution method. In case of using an LPF-HA-containing solution, from which F-HA is previously removed, the elution is carried out by using a buffer having such a salt concentration gradient as a specific conductivity of 0.5→300 ms/cm (for example, a 0.02 M McIlvaine's buffer (pH 5.2) having a sodium chloride concentration gradient of 0→4.0 M) to obtain an LPF-HA-containing fraction, by which a highly purified LPF-HA can be obtained.

According to the above purification method, the purification degree of LPF-HA becomes several ten folds and further the recovery rate of LPF-HA reaches to from more than 80 % to almost 100 %. Beside, the purified LPF-HA has so high specific activity as $0.8 - 0.9 \times 10^5$ LPF-Hp-ELISA unit/mg protein, and further, forms a single band in a polyacrylamide disc electrophoresis analysis (pH 4.5), which means that B. pertussis endotoxin is almost completely removed.

Thus, according to the above purification method, the desired LPF-HA can be isolated from the starting culture of B. pertussis in a high yield and high purity with very simple operation, and the chromatography

EP 0 175 841 B1

adsorbent can be prepared in a low cost and also can be used repeatedly without deterioration, and hence the method is excellent from economical viewpoint. Accordingly, this second purification method is very excellent as an industrial method for production of a highly purified LPF-HA. If necessary, the purification may be combined with conventional purification methods, such as sucrose density gradient ultracentrifugation, ion exchange chromatography, etc.

The purified LPF-HA thus obtained is very pure and does not contain other proteins, lipids, saccharides, etc., and further, endotoxin is almost completely removed, and hence, it can be used as a starting material for producing the final vaccine. For preparing a vaccine, the purified LPF-HA is used after being converted into a toxoid by a conventional method using formalin in the presence or absence of an amino acid.

The pertussis component vaccine of the present invention can be prepared by simply mixing the highly pure F-HA or a toxoid thereof and a toxoid of the highly pure LPF-HA in a ratio of amounts of both components which can give a minimum amount of an antigen necessary for human body. The conversion of LPF-HA into a toxoid thereof may be done either before or after it is mixed with F-HA. For instance, formalin is added to LPF-HA or a mixture thereof with F-HA in an amount of 0.1 to 1.0 %, and the mixture is allowed to stand at 22° to 40°C for 7 to 35 days, and thereafter, formalin is added thereto in an amount of 0.2 %, and the mixture is allowed to stand at room temperature for 1 to 3 days, optionally with shaking in some occasion, by which the toxicity or LPF-HA is lowered. After confirming the lowering of toxicity, the mixture is regulated to a suitable protein content, usually the final protein nitrogen content of 10 to 20 $\mu$g/ml. Thereafter, the mixture is optionally mixed with a conventional adjuvant such as aluminum hydroxide or aluminum phosphate, and further a stabilizer such as gelatin or glucose and also a perservative such as thimerosal is added thereto and the pH is regulated to the range of 5.4 to 7.4 to give the desired pertussis component vaccine. When LPF-HA is alone previously converted into a toxoid, it is mixed with the highly pure F-HA or a toxoid thereof.

The combined vaccine can be prepared by mixing the pertussis component vaccine with diphtheria toxoid (the final concentration: 30 Lf/ml) and tetanus toxoid (the final concentration: 5 Lf/ml).

The vaccines thus obtained may be lyophilized to obtain the product.

The present invention is illustrated by the following Preparations and Examples, but should not be construed to be limited thereto.

Preparation 1

To pyridine (600 ml) is added dropwise chlorosulfonic acid (117 g) at below 0°C. After the addition, the mixture is heated to 65 - 70°C. To the mixture is added crystalline cellulose gel (Cellulofine GC-15, manufactured by Chisso Corp.) (80 g), and the mixture is stirred at 65 - 70°C for 3 hours. After the reaction, the reaction mixture is cooled and neutralized with 10 % aqueous sodium hydroxide. The gel thus obtained is separated by filtration and washed well with 0.01 M phosphate buffer-aqueous sodium chloride mixture to give a cellulose sulfate gel.

The Cellulofine GC-15 sulfate gel obtained in the same manner as described above is packed within a column (400 mmø x 130 mm), and this is equilibrated with 0.2 M sodium chloride-added 0.01 M phosphate buffer (pH 7.6, specific conductivity: about 17.5 mS/cm. A supernatant (specific conductivity: about 17.5 mS/cm, pH 7.6; 300 liters) of a fermenter culture of B. pertussis phase I Tohama strain is passed through the column. After washing well the column with the same buffer solution as above to remove contaminants, the adsorbed material is eluted with 1.5 M sodium chloride-added phosphate buffer solution (pH 7.6) to give a fraction containing F-HA (30 liters).

The analytical data of the supernatant of culture, the fraction passed through, and the fraction containing purified F-HA are shown in Table 1.

The recovery rate of F-HA was 95 %, the degree of purification (specific activity of the fraction of the purified F-HA/specific activity of the supernatant of culture) was 23 times. Besides, the LPF-HA activity of the fraction of the purified F-HA was less than 5.0 LPF-Hp-ELISA unit/ml.

9

## Table 1

| Analytical items | Samples | | |
|---|---|---|---|
| | Supernatant of culture (starting material) | Fraction passed through | Fraction of purified F-HA |
| Amount of sample (ml) | 300,000 | 350,000 | 30,000 |
| Content of F-HA (1) | 2,400 | 2.0 | 22,800 |
| Content of LPF-HA (2) | 1,800 | 1,600 | Less than 5.0 |
| Content of HA (3) | 1,024 | 64 | 10,000 |
| Content of protein (mg/ml) (4) | 0.46 | 0.34 | 0.19 |
| Specific activity of F-HA (5) | $5.2 \times 10^3$ | 4.5 | $1.2 \times 10^5$ |
| Pyrogen test in rabbit (Total in three rabbits, °C) (6) | 6.7 | 6.4 | 1.2 |

[Notes]: (1) It is shown in F-HA-Ab-ELISA unit/ml.

(2) It is shown in LPF-Hp-ELISA unit/ml.

(3) It is shown in HA unit/ml.

(4) It is shown as a protein content when calculated as protein nitrogen measued by Kjeldahl method x 6.25.

(5) It is shown in F-HA-Ab-ELISA unit/mg protein.

(6) It was done in accordance with the method described in Minimum Requirement of Biological Products, Ministry of Health and Welfare, Japan, #287, 1981, wherein the test sample was diluted until protein content of 6.25 $\mu$g/ml.

Preparation 2

To pyridine (600 ml) is added dropwise chlorosulfonic acid (117 g) at below 0° C. After the addition, the mixture is heated to 65 - 70° C. To the mixture is added crystalline cellulose (Abicel for chromatography, manufactured by Asahi Kasei) (80 g), and the mixture is stirred at 65 - 70° C for 4 hours. After the reaction, the reaction mixture is cooled and then neutralized with 10 % aqueous sodium hydroxide. The gel thus obtained is separated by filtration and washed well with 0.01 M phosphate buffer-aqueous sodium chloride mixture to give a cellulose sulfate gel.

The Abicel sulfate gel obtained in the same manner as described above is packed within a column (400 mmø x 130 mm), and this is equilibrated with 0.14 M sodium chloride-added 0.01 M phosphate buffer (pH 7.6). A supernatant (specific conductivity: about 15 m$^S$/cm, pH 7.6; 300 liters) of a fermenter culture of B. pertussis phase I Tohama strain is passed through the column. After washing well the column with the same buffer solution as above to remove contaminants, the adsorbed material is eluted with 1.5 M sodium chloride-added phosphate buffer solution (pH 7.6) to give a fraction containing F-HA (30 liters).

The analytical data of the supernatant of culture, the fraction passed through, and the fraction containing purified F-HA are shown in Table 2.

The recovery rate of F-HA was 75 %, the degree of purification was 14 times.

## Table 2

| Analytical items | Samples | | |
|---|---|---|---|
| | Supernatant of culture (starting material) | Fraction passed through | Fraction of purified F-HA |
| Amount of sample (ml) | 300,000 | 350,000 | 30,000 |
| Content of F-HA (1) | 3,200 | 5.0 | 24,000 |
| Content of LPF-HA (2) | 1,900 | 1,600 | Less than 5.0 |
| Content of HA (3) | 1,024 - 2,048 | 64 | 10,000 |
| Content of protein (mg/ml) (4) | 0.51 | 0.34 | 0.27 |
| Specific activity of F-HA (5) | $6.3 \times 10^3$ | $1.4 \times 10^1$ | $0.9 \times 10^5$ |
| Pyrogen test in rabbit (Total in three rabbits, °C) (6) | 6.9 | 6.9 | 0.9 |

[Notes]: The notes in (1), (2), (3), (4), (5), and (6) are the same as those in Table 1.

Preparation 3

To pyridine (500 ml) is added dropwise chlorosulfonic acid (82 g) at 0° - 5°C. After the addition, the mixture is heated to 65 - 70°C. To the mixture is added crystalline cellulose gel (Cellulofine GH-25, manufactured by Chisso Corp.) (80 g), and the mixture is stirred at 65 - 70°C for 4 hours. After the reaction, the reaction mixture is cooled and then neutralized by gradually adding thereto 10 % aqueous sodium hydroxide. The gel thus obtained is separated by filtration and washed well with 0.01 M phosphate buffer-aqueous sodium chloride mixture (pH 7.2) to give a cellulose sulfate gel.

The cellulose sulfate gel obtained in the same manner as described above is packed within a column (400 mmø x 130 mm), and this is equilibrated with 0.2 M sodium chloride-added 0.01 M phosphate buffer (pH 7.6). A supernatant (specific conductivity: about 17.5 mS/cm, pH 7.6; 300 liters) of the same rot of a fermenter culture of B. pertussis phase I Tohama strain as used in Preparation 1 is passed through the column. After washing well the column with the same buffer solution as above to remove contaminants, the adsorbed material is eluted with 1.5 M sodium chloride-added phosphate buffer solution (pH 7.6) to give a fraction containing F-HA (30 liters).

The analytical data of the supernatant of culture, the fraction passed through, and the fraction containing purified F-HA are shown in Table 3.

The recovery rate of F-HA was 93.8 %, the degree of purification was 25 times.

According to the method as described in Minimum Requirement of Biological Products, "Pertussis Vaccine" (cf. Notification of the Pharmaceutical Affairs Bureau, Ministry of Health and Welfare, Japan, #287, 1981), the purified product was subjected to test for mouse body weight-decreasing toxicity, test for mouse leucocyte-increasing toxicity, test for freedom from heat-labile toxin, and test for mouse histamine sensitizing toxicity. As a result, in all tests, the purified product was the same as the control (a physiological saline solution was used), which means that no side effect was observed.

## Table 3

| Analytical items | Samples | | |
|---|---|---|---|
| | Supernatant of culture (starting material) | Fraction passed through | Fraction of purified F-HA |
| Amount of sample (ml) | 300,000 | 350,000 | 3,000 |
| Content of F-HA (1) | 2,400 | 2.0 | 22,440 |
| Content of LPF-HA (2) | 1,800 | 1,500 | Less than 5.0 |
| Content of HA (3) | 1,024 | 64 | 10,000 |
| Content of protein (mg/ml) (4) | 0.46 | 0.30 | 0.17 |
| Specific activity of F-HA (5) | $5.2 \times 10^3$ | 6.7 | $1.3 \times 10^5$ |
| Pyrogen test in rabbit (Total in three rabbits, °C) (6) | 6.7 | 6.5 | 1.0 |

[Notes]:   The notes in (1), (2), (3), (4), (5), and (6) are the same as those in Table 1.

Preparation 4

Sodium dextran sulfate (5 g) is dissolved in 0.5 M aqueous sodium carbonate (200 ml), and thereto is added Cepharose CL-4B (agarose gel, manufactured by Pharmacia Fine Chemicals, Sweden) (20 ml) which is equilibrated by 0.5 M aqueous sodium carbonate, and the mixture is gently stirred. To the mixture is added with stirring a solution of cyano bromide (10 g) in distilled water (100 ml). The mixture is maintained for 15 minutes while keeping at pH 11 by adding 5 M aqueous sodium hydroxide. Thereafter, the mixture is stirred at room temperature for 17 hours, while allowing to lower the pH value. After the reaction, the reaction mixture is filtered with a glass filter, and the gel thus obtained is washed well with 0.15 M sodium chloride-added phosphate buffer (pH 7.2) to give dextran sulfate agarose gel (20 ml).

The dextran sulfate agarose gel obtained in the same manner as described above is packed within a column (16 mmø x 100 mm), and this is equilibrated with 0.2 M sodium chloride-added 0.01 M phosphate buffer (pH 7.6, specific conductivity: about 17.5 mS/cm). A supernatant (specific conductivity; about 17.5 mS/cm, pH 7.6; 800 ml) of a fermenter culture of B. pertussis phase I Tohama strain is passed through the column. After washing well the column with the same buffer solution as above and further 0.20 M sodium chloride-added phosphate buffer (pH 7.6, specific conductivity: about 17.5 mS/cm) to remove contaminants, the adsorbed material is eluted with 1.5 M sodium chloride-added phosphate buffer solution (pH 7.8, specific conductivity: about 120 ms/cm) to give a fraction containing F-HA (29 ml).

The analytical data of the supernatant of culture, the fraction passed through, and the fraction containing purified F-HA are shown in Table 4.

The recovery rate of F-HA was 90 %, the degree of purification was 17 times.

## Table 4

| Analytical items | Samples | | |
|---|---|---|---|
| | Supernatant of culture (starting material) | Fraction passed through | Fraction of purified F-HA |
| Amount of sample (ml) | 800 | 1,000 | 29 |
| Content of F-HA (1) | 2,400 | 4.0 | 59,600 |
| Content of LPF-HA (2) | 1,800 | 1,400 | Less than 5.0 |
| Content of HA (3) | 1,024 | 64 | 25,600 - 51,200 |
| Content of protein (mg/ml) (4) | 0.46 | 0.33 | 0.66 |
| Specific activity of F-HA (5) | $5.2 \times 10^3$ | $1.2 \times 10^1$ | $0.9 \times 10^5$ |
| Pyrogen test in rabbit (Total in three rabbits, °C) (6) | 6.7 | 6.4 | 1.0 |

[Notes]: The notes in (1), (2), (3), (4), (5), and (6) are the same as those in Table 1.

Preparation 5

To pyridine (200 ml) is added dropwise chlorosulfonic acid (11 ml) at below 0° C. After the addition, the mixture is heated to 65 - 70° C. To the mixture is added epichlorohydrin-crosslinked dextran (Sephadex G-50, manufactured by Pharmacia) (7.5 g), and the mixture is stirred at 65 - 70° C for 4 hours. After the reaction, the reaction mixture is cooled and then neutralized with aqueous sodium hydroxide. The gel thus obtained is separated by filtration and washed well with 0.01 M phosphate buffered saline solution to give a crosslinked dextran sulfate.

The Sephadex G-50 sulfate gel obtained in the same manner as described above is packed within a column (16 mmø x 100 mm), and this is equilibrated with 0.2 M sodium chloride-added 0.01 M phosphate buffer (pH 7.6, specific conductivity: about 17.5 mS/cm). A supernatant (specific conductivity: about 17.5 mS/cm, pH 7.6; 800 ml) of a fermenter culture of B. pertussis phase I Tohama strain is passed through the column. After washing well the column with the same buffer solution as above to remove contaminants, the adsorbed material is eluted with 1.5 M sodium chloride-added phosphate buffer solution (pH 7.6) to give a fraction containing F-HA (30 ml).

The analytical data of the supernatant of culture, the fraction passed through, and the fraction containing purified F-HA are shown in Table 5.

The recovery rate of F-HA was 93.8 %, the degree of purification was about 20 times.

## Table 5

| Analytical items | Samples | | |
|---|---|---|---|
| | Supernatant of culture (starting material) | Fraction passed through | Fraction of purified F-HA |
| Amount of sample (ml) | 800 | 1,000 | 30 |
| Content of F-HA (1) | 2,800 | 5.0 | 70,000 |
| Content of LPF-HA (2) | 150 | 125 | Less than 5 |
| Content of HA (3) | 1,024 | 8 | 64,000 |
| Content of protein (mg/ml) (4) | 0.46 | 0.33 | 0.58 |
| Specific activity of F-HA (5) | $6.1 \times 10^3$ | $1.5 \times 10^1$ | $1.2 \times 10^5$ |
| Pyrogen test in rabbit (Total in three rabbits, °C) (6) | 7.1 | 6.9 | 0.7 |

[Notes]: The notes in (1), (2), (3), (4), (5), and (6) are the same as those in Table 1.

Preparation 6

The fraction passed through (64 HA unit/ml, 1700 LPF-Hp-ELISA unit/ml, 0.46 mg protein/ml, pH 7.6) which is obtained in the adsorption of F-HA-containing solution with cellulose sulfate gel in the above Preparation 1 is used as the starting material.

The starting material is passed through a hydroxyapatite column equilibrated with 0.01 M phosphate buffer (pH 6.0) according to the method disclosed in "L. I. Irons et al., Biochim. Biophys. Acta, 580 , 175-185, 1979", by which LPF-HA is adsorbed. The adsorbed material is eluted with 0.5 M sodium chloride-containing 0.1 M phosphate buffer (pH 7.0) to obtain a protein fraction (partially purified LPF-HA) . The protein fraction thus obtained is subjected to affinity chromatography using as a ligand haptoglobinsepharose, and the adsorbed material is eluted with 0.05 M phosphate buffer (pH 7.6) which contains 0.5 M sodium chloride and 3 M potassium thiocyanate, by which there is recovered purified LPF-HA showing analytical data as shown in Table 6 (the recovery rate: 53 %).

Table 6

| Samples | Analytical items | | | | | | | | |
|---|---|---|---|---|---|---|---|---|---|
| | Amount of sample | Content of LPF-HA[1] | Content of protein (mg/ml)[2] | Content of HA[3] | Specific activity of LPF-HA[4] | Purity (%)[5] | Yield (%) | Content of F-HA[6] | Pyrogen test in rabbit (total in three rabbits, °C)[7] |
| Starting material | 5,000 | 1,700 | 0.34 | 64 | 5,000 | - | 100 | 2.0 | 6.5 |
| Purified LPF-HA | 100 | 45,000 | 0.64 | 512 | 70,300 | 70 | 53.0 | 0 | 1.2 |

(1) It is shown in LPF-Hp-ELISA unit/ml.

(2) It is shown as a protein content when calculated as protein nitrogen measued by Kjeldahl method x 6.25.

(3) It is shown in HA unit/ml.

(4) It is shown in LPF-Hp-ELISA unit/mg protein.

(5) It was calculated by polyacrylamide gel electrophoresis - densitometer analysis.

(6) It is shown in F-HA-Ab-ELISA unit/ml.

(7) It was done in accordance with the method described in Minimum Requirement of Biological Products, Ministry of Health and Welfare, Japan, #287, 1981, wherein the test sample was diluted until protein content of 6.25 $\mu$g/ml, followed by inactivating.

EP 0 175 841 B1

Preparation 7

Preparation of human ceruloplasmin:

Cohn's Fraction IV-1 obtained by alcohol fraction of human plasma is used as the starting material. The starting material is subjected to the purification method of Morell et al. [cf. J. Biol. Chem., 224 , 3494 (1967)] to separate human ceruloplasmin.

That is, the Cohn's Fraction IV-1 is subjected to an ion exchange chromatography with DEAE-Sepharose CL-6B, and it is subjected to an affinity chromatography with a hemoglobin-Sepharose gel in order to remove the possible contamination of haptoglobin. Thereafter, the solution is salted out with ammonium sulfate and then dialyzed against 0.025 M acetate buffer (pH 5.25) to obtain crystalline human ceruloplasmin.

Preparation of denatured ceruloplasmin:

The crystalline ceruloplasmin obtained above is denatured in a similar manner to the procedure described in Morell et al., Science, 127 , 588 (1963) to prepare the following various denatured ceruloplasmins.

(1) Ceruloplasmin denatured with sodium cyanide:

A 1 w/v % aqueous solution of crystalline ceruloplasmin (50 ml) is dialyzed against a phosphate buffer (pH 7.4, ionic strength: 0.2) containing 0.05 M sodium cyanide (5.0 liters) at 4° C for 12 hours to give a sodium cyanide-denatured ceruloplasmin which looses 90 % of oxidase activity.

(2) Heat treatment of sodium cyanide-denatured ceruloplasmin:

A 1 w/v % aqueous solution of sodium cyanide-denatured ceruloplasmin is prepared in the same manner as described in the above (1). The solution (50 ml) is dialyzed against 0.1 M phosphate buffer (pH 7.4) (5.0 liters). After the dialysis, the denatured ceruloplasmin-containing solution is heated at 60° C for 10 hours.

(3) Ceruloplasmin denatured with L-ascorbic acid:

A 1 w/v % aqueous solution of crystalline ceruloplasmin is dialyzed against an acetate buffer (pH 5.2, ionic strength: 1.2) (5.0 liters) containing L-ascorbic acid (5 mg/ml) at 4° C for 36 hours to give an L-ascorbic acid-denatured ceruloplasmin which show 65.5 % oxidase activity.

(4) Ceruloplasmin denatured by heat treatment:

A 0.1 M phosphate buffer (pH 7.5) (100 ml) containing 5 w/v % of a crystalline ceruloplasmin is heat-treated at 60° C for 15 hours.

(5) Heat-treated ceruloplasmin denatured with L-ascorbic acid:

Cohn's Fraction IV-1 obtained by alcoholic fractionation of human plasma is heated on a bath at 60° C for 10 hours. The solution is treated in the same manner as described by Morell et al. [cf. Science, 127 , 568 (1963)] to give a heat-treated crystalline ceruloplasmin.

The heat-treated crystalline ceruloplasmin thus obtained is treated in the same manner as described in the above (3) to give a heat-treated ceruloplasmin denatured with L-ascorbic acid.

Preparation of affinity gel:

The above various denatured ceruloplasmins (as a ligand) are subjected to coupling reaction with CNBr-activated Sepharose 4B (manufactured by Pharmacia) to prepare affinity gels in the following manner.

CNBr-activated Sepharose 4B (1.5 g) is swollen by dipping in 1.0 mM hydrochloric acid (3.0 liters) for 15 minutes, and then 1.0 mM hydrochloric acid is removed by suction on a glass filter to give a swollen Sepharose gel (5.25 ml).

Separately, a ligand (protein amount: 150 mg) is dissolved in 0.1 M sodium carbonate buffer (pH 8.3, 75 ml) containing 0.5 M sodium chloride, and thereto is added the above-prepared swollen Sepharose gel (5.25 ml). The mixture is gently stirred at room temperature for 2 hours to complete the coupling reaction. After the coupling reaction, the reaction mixture is washed with the same sodium carbonate buffer as above (150 ml) four times, and thereto is added 1.0 M ethanolamine (pH 8.0, 150 ml), and the mixture is again reacted with gently stirring for 2 hours. After completion of the reaction, the reaction mixture is washed with the same sodium carbonate buffer (150 ml) four times to remove ethanolamine. The resulting gel is washed with 0.1 M acetate buffer (pH 8.0) containing 1.0 M sodium chloride (150 ml) three times, and further with 0.1 M borate buffer (pH 8.0) containing 1.0 M sodium chloride (150 ml) three times. The washing with the acetate buffer and the borate buffer are mutually repeated each three times to give various denatured ceruloplasmin-Sepharose affinity gels.

Purification of LPF-HA by column method:

The affinity gel (20 ml) prepared above is packed in a column (28 mmø x 32 mmø), and the same starting material (5.0 liters) as used in the above Preparation 6 is passed through the column at room temperature at a flow rate of 150 ml/cm²/hour. Thereafter, a 0.1 M phosphate buffer (pH 7.0, 1,500 ml) containing 1.0 M sodium chloride is passed through the column at the same flow rate as above to wash the column.

After the washing, an eluent (100 ml) consisting of 0.1 M phosphate buffer (pH 7.5) containing 1.0 M sodium chloride and 3.0 M sodium thiocyanate is passed through the column at a flow rate of 35.0 ml/cm²/hour in order to elute LPF-HA.

There are disclosed in Table 7 the analytical data of the LPF-HA fraction and experimental data in the cases of using affinity gels wherein the various denatured ceruloplasmins are used as the ligand.

As is clear from the results, in case of using various denatured ceruloplasmins as the ligand, the LPF-HA obtained has a high purity and a high specific activity.

Table 7

| Samples | Analytical items | | | | | | | | |
|---|---|---|---|---|---|---|---|---|---|
| | Amount of sample | Content of LPF-HA [1] | Content of protein (mg/ml) [2] | Content of HA [3] | Specific activity of LPF-HA [4] | Purity (%) [5] | Yield (%) | Content of F-HA [6] | Pyrogen test in rabbit (total in three rabbits, °C) [7] |
| Starting material | 5,000 | 1,700 | 0.34 | 64 | 5,000 | – | (100) | 2.0 | 6.5 |
| LPF-HA purified with NaCN-denatured ceruloplasmin | 100 | 78,200 | 0.67 | 1,024 | 116,700 | 92.3 | 92.0 | 0 | 0.5 |
| LPF-HA purified with NaCN-denatured, heat-treated ceruloplasmin | 100 | 76,500 | 0.65 | 1,024 | .117,700 | 93.0 | 90.0 | 0 | 0.6 |

– to be continued –

[Note]: The meanings of (1), (2), (3), (4), (5), (6) and (7) are the same as in Table 6.

EP 0 175 841 B1

Table 7     (Continued)

| Samples | Analytical items | | | | | | | | |
|---|---|---|---|---|---|---|---|---|---|
| | Amount of sample | Content of LPF-IIA [1] | Content of protein (mg/ml) [2] | Content of IIA [3] | Specific activity of LPF-IIA [4] | Purity (%) [5] | Yield (%) | Content of F-IIA [6] | Pyrogen test in rabbit (total in three rabbits, °C) [7] |
| LPF-IIA purified with l-ascorbic acid denatured ceruloplasmin | 100 | 79,900 | 0.68 | 1,024 | 117,500 | 93.1 | 94.0 | 0 | 0.7 |
| LPF-IIA purified with heat-treated ceruloplasmin | 100 | 78,200 | 0.65 | 1,024 | 120,300 | 94.6 | 92.0 | 0 | 0.6 |
| LPF-IIA purified with heat-treated, l-ascorbic acid denatured ceruloplasmin | 100 | 84,200 | 0.70 | 1,024 | 120,300 | 95.3 | 99.0 | 0 | 0.4 |

EP 0 175 841 B1

Preparation 8

The Cellulofine GC-15 sulfate gel obtained in the same manner as described in the above Preparation 1 is packed within a column (40 mmø x 200 mm), and therethrough is passed distilled water (1.0 liter). A supernatant (5,000 ml) of a fermenter culture of B. pertussis phase I Tohama strain is diluted with distilled water in 10 folds and the diluted solution (specific conductivity: about 1.5 mS/cm) is passed through the column. After washing well the column with 0.02 M McIlvaine's buffer (pH 5.2, about 50,000 ml), the adsorbed material is eluted with 0.02 M sodium chloride-added McIlvaine's buffer (specific conductivity: about 2.0 mS/cm, pH 5.2) in the concentration gradient of sodium chloride of 0→4.0 M, whereby fractions (each about 20 ml) are collected and then the fraction containing LPF-HA (about 130 ml) is pooled.

The analitical data and experimental data of the starting material and the purified LPF-HA fraction are shown in Table 8.

EP 0 175 841 B1

Table 8

| Samples | Analytical items | | | | | | | |
|---|---|---|---|---|---|---|---|---|
| | Amount of sample | Con-[1] tent of LPF-HA | Con-[2] tent of protein (mg/ml) | Con-[3] tent of HA | Speci-[4] fic activity of LPF-HA | Purity[5] (%) | Yield (%) | Con-[6] tent of F-HA | Pyrogen[7] test in rabbit (total in three rabbits, °C) |
| Starting material | 5,000 | 100 | 0.250 | 1,024 | 400 | – | (100) | 2,400 | 6.9 |
| Purified LPF-HA fraction | 130 | 3,200 | 0.037 | 16 | 86,000 | 95.0 | 84 | 5.0 | 0.9 |

[Note]: The meanings of (1), (2), (3), (4), (5), (6), and (7) are the same as in Table 6.

The dextran sulfate-agarose gel obtained in the same manner as described in the above Preparation 4 is packed within a column (40 mmø x 200 mm), and therethrough is passed distilled water (1,000 ml). The same supernatant (5,000 ml) as used in the above Preparation 8 is diluted with distilled water in 7 folds and the diluted solution (specific conductivity: about 2.0 mS/cm) is passed through the column. After washing well the column with 0.02 M McIlvaine's buffer (pH 5.2, about 20,000 ml), the adsorbed material is eluted with 0.02 M sodium chloride-added McIlvaine's buffer (specific conductivity: about 2.0 mS/cm, pH 5.2) in the concentration gradient of sodium chloride of 0→4.0 M, whereby fractions (each about 20 ml) are collected and then the fraction containing LPF-HA (about 200 ml) is pooled.

The analitical data and experimental data of the starting material and the purified LPF-HA fraction are shown in Table 9.

Table 9

| Samples | Analytical items | | | | | | | | |
|---|---|---|---|---|---|---|---|---|---|
| | Amount of sample | Content of LPF-HA [1] | Content of protein (mg/ml) [2] | Content of HA [3] | Specific activity of LPF-HA [4] | Purity (%) [5] | Yield (%) | Content of F-HA [6] | Pyrogen test in rabbit (total in three rabbits, °C) [7] |
| Starting material | 5,000 | 100 | 0.250 | 1,024 | 400 | – | (100) | 2,400 | 6.9 |
| Purified LPF-HA fraction | 200 | 2,200 | 0.029 | 16 | 75,000 | 92.0 | 85 | 5.0 | 0.7 |

[Note]: The meanings of (1), (2), (3), (4), (5), (6), and (7) are the same as in Table 6.

Preparation 10

.To pyridine (200 ml) is added dropwise chlorosulfonic acid (11 ml) at below 0°C. After the addition, the mixture is heated to 65 - 70°C. To a mixture of pyridine - chlorosulfonic acid is added crosslinked agarose gel (Sepharose CL-6B, manufactured by Pharmacia) (30 ml) which is impregnated by pyridine, and the mixture is reacted at 65 - 70°C for 4 hours. After the reaction, the reaction mixture is cooled and neutralized with aqueous sodium hydroxide. The gel thus obtained is separated by filtration and washed well with 0.01 M phosphate buffered saline solution to give a crosslinked agarose sulfate (23 ml).

The crosslinked agarose sulfate gel obtained above is packed within a column (40 mmø x 200 mm), and therethrough is passed distilled water (1,000 ml). The same supernatant (5,000 ml) as used in the above Preparation 8 is diluted with distilled water in 9 folds and the diluted solution (specific conductivity: about 2.0 m$^S$/cm) is passed through the column. After washing well the column with 0.02 M McIlvaine's buffer (pH 5.2, about 20,000 ml), the adsorbed material is eluted with 0.02 M sodium chloride-added McIlvaine's buffer (specific conductivity: about 2.0 m$^S$/cm, pH 5.2) in the concentration gradient of sodium chloride of 0→4.0 M, whereby fractions (each about 20 ml) are collected and then the fraction containing LPF-HA (about 200 ml) is pooled. The analitical data and experimental data of the starting material and the purified LPF-HA fraction are shown in Table 10.

Table 10

| Samples | Analytical items | | | | | | | | |
|---|---|---|---|---|---|---|---|---|---|
| | Amount of sample | Content of LPF-HA [1] | Content of protein (mg/ml) [2] | Content of HA [3] | Specific activity of LPF-HA [4] | Purity (%) [5] | Yield (%) | Content of F-HA [6] | Pyrogen test in rabbit (total in three rabbits, °C) [7] |
| Starting material | 5,000 | 100 | 0.250 | 1,024 | 400 | – | (100) | 2,400 | 6.9 |
| Purified LPF-HA fraction | 200 | 2,050 | 0.026 | 16 | 80,000 | 95.0 | 82 | Less than 5 | 0.9 |

[Note]: The meanings of (1), (2), (3), (4), (5), (6), and (7) are the same as in Table 6.

EP 0 175 841 B1

Examples 1 to 7

For convenience, the purified F-HAs obtained in Preparations 1, 2, 3, 4 and 5 are referred to as Samples a, b, c, d, and e, respectively, and the purified LPF-HAs obtained in Preparations 6, 7, 8, 9 and 10 are referred to as Samples V, W, X, Y, and Z, respectively.

By using these samples, various pertussis component vaccines are prepared in the following manner. The above samples are sterilized by filtering with a membrane filter (0.45 ) in the final step of the purification.

To each Samples V, W, X, Y, and Z are added gelatin (final concentration: 0.02 v/v %) and Tween 80 (final concentration: 0.05 v/v %), and further added formalin (final concentration: 0.6 v/v %), and the mixture is detoxified by heating at 40°C for 10 days to give each LPF-HA toxoid. Formalin is removed by dialyzing against a phosphate buffer (pH 6.7) at 4°C for 2 days.

Separately, Samples a, c and e are each formalinzed with formalin (final concentration: 0.02 v/v %) at 40°C overnight in order to remove the slightly remained LPF activity, by which F-HA toxoid is obtained. Formalin is removed therefrom in the same manner as described above. Samples b and d are used for the preparation of vaccines as they stand (i.e. without forming into toxoid).

These F-HA Samples b and d, and toxoids of F-HA Samples a, c and e are mixed with the toxoids of LPF-HA Samples V, W, X, Y, and Z in various combinations as shown in Table 11, and to each mixture are added A1(OH)$_3$ (final concentration as aluminum: about 0.2 mg/ml) and thimerosal (final concentration: 0.01 v/v %), and the mixture is regulated to the final pH 6.7 - 6.8 to give seven kinds of precipitated pertussis component vaccine.

## Table 11

| Example No. | Mixing ratio * | Final protein concentration ($\mu$g/ml) |
|---|---|---|
| 1 | a : V = 50 : 50 | 100 |
| 2 | a : W = 50 : 50 | 100 |
| 3 | a : X = 50 : 30 | 80 |
| 4 | b : Y = 20 : 50 | 70 |
| 5 | c : Z = 50 : 10 | 60 |
| 6 | d : W = 50 : 25 | 75 |
| 7 | e : W = 50 : 40 | 90 |

*) The numeral is shown by the final content of protein.

All vaccines of Examples 1 to 7 were subjected to tests of general properties, toxicity in mice and activity in mice, in accordance with Minimum Requirement of Biological Products, Ministry of Health and Welfare, Japan, #287, 1981. As a result, all properties thereof were satisfactory as shown in Table 12.

Table 12

| Test item | 1 | 2 | 3 | 4 | 5 | 6 | 7 |
|---|---|---|---|---|---|---|---|
| | | | | Example No. | | | |
| Content of protein nitrogen ($\mu$gN/ml) | 16.1 | 16.3 | 12.8 | 11.5 | 9.3 | 12.0 | 14.5 |
| Sterility test | Passed | Passed | Passed | Passed | Passed | Passed | Passed |
| Staining test | Passed | Passed | Passed | Passed | Passed | Passed | Passed |
| Hydrogen ion concentration | 6.7 | 6.7 | 6.7 | 6.7 | 6.8 | 6.7 | 6.7 |
| Content of aluminum ($\mu$g/ml) | 0.201 | 0.205 | 0.207 | 0.201 | 0.200 | 0.205 | 0.204 |
| Content of thimerosal (w/v %) | 0.009 | 0.009 | 0.010 | 0.011 | 0.009 | 0.009 | 0.009 |
| Content of formaldehyde (w/v %) | 0.001 | 0.001 | 0.001 | 0.001 | 0.001 | 0.001 | 0.001 |
| Test for freedom from heat-labile toxin | Passed | Passed | Passed | Passed | Passed | Passed | Passed |
| Test for mouse body weight-decreasing toxicity (BWDU/ml) | 7.73 (3.59-16.65) | 6.99 (3.48-14.04) | 11.21 (5.77-21.78) | 4.79 (2.31-9.95) | 6.71 (3.33-13.52) | 7.50 (3.76-14.98) | 5.83 (2.86-11.89) |

Table 12    (Continued)

| Test item | Example No. | | | | | | |
|---|---|---|---|---|---|---|---|
| | 1 | 2 | 3 | 4 | 5 | 6 | 7 |
| Test for mouse leucocyte-increasing toxicity (LPU/ml) | 0.43 (0.23–0.80) | 0.56 (0.42–0.75) | 0.59 (0.44–0.77) | 0.59 (0.44–0.80) | 0.53 (0.39–0.72) | 0.61 (0.46–0.81) | 0.48 (0.35–0.65) |
| Test for mouse histamine sensitizing toxicity (HSU/ml) | 0.55 (0.25–1.23) | 0.38 (0.23–0.62) | 0.33 (0.20–0.54) | 0.05 (0.02–0.08) | 0.16 (0.10–0.28) | 0.14 (0.08–0.23) | 0.19 (0.12–0.32) |
| Pyrogen test in rabbit (total temp.°C in two) | 0.8 | 1.2 | 1.1 | 0.8 | 0.9 | 0.6 | 0.7 |
| Test for freedom from abnormal toxicity: | | | | | | | |
| In mice | Passed | Passed | Passed | Passed | Passed | Passed | Passed |
| In guinea pig | Passed | Passed | Passed | Passed | Passed | Passed | Passed |
| Potency test (IPU/ml) | 31.5 | 44.4 | 32.9 | 33.9 | 21.7 | 27.8 | 32.8 |

EP 0 175 841 B1

Example 8

The F-HA toxoid a and LPF-HA toxoid W prepared in Examples 1 and 7 (each final concentration: 50μg protein/ml) are mixed with diphtheria toxoid and tetanus toxoid (final concentration: 33 Lf/ml and 5 Lf/ml, respectively) and further with Al(OH)$_3$, gelatine and thimerosal (final concentration: 0.2 mgAl/ml, 0.02 w/v %, and 0.01 %, respectively), and the mixture is regulated to the final pH of 6.7 to give a precipitated purified pertussis-diphtheria-tetanus vaccine.

The properties of this vaccine were tested in accordance with Minimum Requirement of Biological Products, Ministry of Health and Welfare, Japan, #287, 1981. As a result, all properties thereof were satisfactory as shown in Table 13. Besides, the lyophilized product of the above vaccine showed the similar results as those before lyophilization.

## Table 13

| Item | Result |
|---|---|
| Content of protein nitrogen ($\mu$gN/ml) | 35.2 |
| Sterility test | Passed |
| Staining test | Passed |
| Hydrogen ion concentration | 6.7 |
| Content of aluminum ($\mu$g/ml) | 0.201 |
| Content of thimerosal (w/v%) | 0.009 |
| Content of formaldehyde (w/v%) | 0.001 |
| Test for freedom from heat-labile toxin | Passed |
| Test for mouse body weight-decreasing toxicity (BWDU/ml) | 9.40 (4.20-17.20) |
| Test for mouse leucocyte increasing toxicity (LPU/ml) | 0.66 (0.33-1.00) |
| Test for mouse histamine sensitizing toxicity (HSU/ml) | 0.07 (0.02-0.13) |
| Pyrogen test in rabbit (total temp.°C in two) | 0.5 |
| Test for freedom from abnormal toxicity: | |
|    In mice | Passed |
|    In guinea pig | Passed |
| Potency test | |
|    Pertussis vaccine (IPU/ml) | 25.8 |
|    Diphtheria toxoid (U/ml) | 52.0 |
|    Tetanus toxoid (U/ml) | 45.0 |

Besides, the vaccines of the present invention were compared with commercially available vaccines in terms of topical reaction (as the side effect) by a mouse sole reaction test.

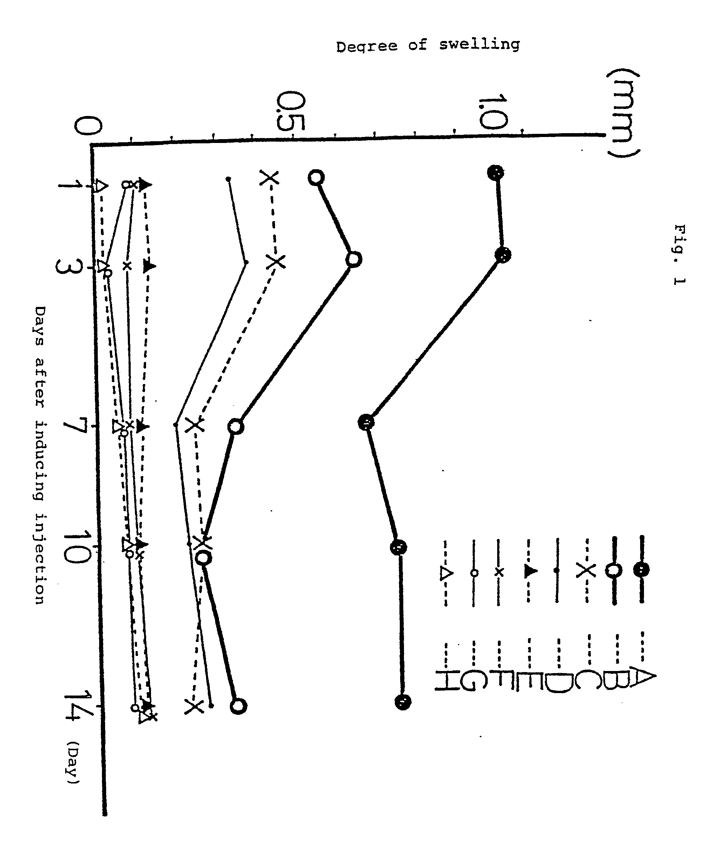

The test was carried out by a modification of the method of Munoz, J.J. et al. [cf. J. Reticuloendotherial Society, 27 , 259-268 (1980)]. That is, dd/F, SPF mice (4 week age, one group: 10 mice) were immunized by intraperitoneal injection of each vaccine (0.5 ml), and after two weeks, the same vaccine (0.025 ml) was subcutaneously innoculated into the footpad or mice (inducing injection). After the innoculation, the increased thickness of the footpad of mice was measured at a fixed interval with Dial thickness guage, and

EP 0 175 841 B1

the average value in each group of 10 mice was shown as the degree of swelling. The results are shown in the accompanying Fig. 1. Fig. 1 shows a graph of plotting of the degree of swelling measured at 1st, 3rd, 7th, 10th and 14th day after the inducing injection of each vaccine.

As is clear from the results, the vaccines (E to G) of the present invention showed significantly lower swelling of the footpad of mice (lower side effect) in comparison with an old product (A) and the commercially available products (B to D) which are accellula vaccine.

The tested vaccines are as follows:

A ... Cell vaccine (a combined vaccine of pertussis and diphtheria toxoid) (an old commercial product)

B ... Precipitated purified pertussis antigen - diphtheria toxoid - tetanus toxoid combined vaccine) (a commercially available product)

C ... The same above (")

D .. The same above (")

E ... The vaccine of Example 1

F ... The vaccine of Example 4

G ... The vaccine of Example 8

H ... PBS (sodium chloride-added phosphate buffer) containing 0.2 mg of aluminum (pH 6.7) (reference)

[Note]: The commercially available products B, C and D are sold by each different companies.

Thus, the present invention can provide improved pertussis component vaccine and combined vaccine of pertussis antigen - diphtheria toxoid - tetanus toxoid which have no side effect such as topical reaction in industrial scale with less expense because the starting F-HA and LPF-HA can be obtained easily and in high yield and high purity.

**Claims**

1. A method for preparing a pertussis component vaccine, which comprises mixing a purified filamentous hemagglutinin (F-HA) or a toxoid thereof and a purified leucocytosis-promoting factor hemagglutinin (LPF-HA) toxoid which are obtained from a culture medium of Bordetella pertussis, said purified F-HA being prepared by contacting an F-HA-containing solution with a cellulose sulfate gel, a polysaccharide gel chemically bound with dextran sulfate or a crosslinked polysaccharide sulfate gel to adsorb the F-HA and eluting the adsorbed F-HA from the gel.

2. The method according to claim 1, wherein the cellulose sulfate gel, polysaccharide gel chemically bound with dextran sulfate or crosslinked polysaccharide sulfate gel is previously equilibrated by treating it with a buffer having a pH of 6.0 to 9.0 and a specific conductivity of 5.0 to 25.0 mS/cm and then subjected to the adsorption of F-HA.

3. The method according to claim 1, wherein the adsorption is carried out under the conditions of a pH of 6.0 to 8.0, a temperature of 0° to 30° C and a specific conductivity of 5.0 to 25.0 mS/cm.

4. The method according to claim 1, wherein the elution of F-HA from the gel is carried out with a buffer having a pH of 5.0 to 10.0 and a specific conductivity of 25.0 to 130 mS/cm.

5. The method according to claim 4, wherein the F-HA-adsorbed gel is washed with a buffer having a pH of 5.0 to 10.0 and a specific conductivity of 5.0 to 25.0 mS/cm before the elution.

6. The method according to claim 1, wherein the cellulose sulfate is a sulfuric acid ester of a crystalline cellulose or a cellulose having crystalline area and non-crystalline area.

7. The method according to claim 1, wherein the polysaccharide gel chemically bound with dextran sulfate is a dextran sulfate-agarose gel, a dextran sulfate-dextran gel, or a dextran sulfate-cellulose gel.

8. The method according to claim 1, wherein the crosslinked polysaccharide sulfate is a crosslinked dextran sulfate, a crosslinked agarose sulfate, or a crosslinked cellulose sulfate.

9. The method according to claim 8, wherein the crosslinked dextran sulfate is an epichlorohydrin-crosslinked dextran sulfate.

10. The method according to claim 8, wherein the crosslinked agarose sulfate is an epichlorohydrin-crosslinked agarose sulfate.

11. The method according to claim 8, wherein the crosslinked cellulose sulfate is an epichlorohydrin-crosslinked cellulose sulfate.

12. The method according to claim 1, wherein the purified LPF-HA is prepared by subjecting an LPF-HA-containing solution to an affinity chromatography using haptoglobin or ceruloplasmin as a ligand to adsorb the LPF-HA and eluting the LPF-HA.

13. The method according to claim 12, wherein the LPF-HA-containing solution is partially purified by treating it with a hydroxyapatite before the affinity chromatography using haptoglobin or ceruloplasmin as a ligand.

14. The method according to claim 1, wherein the purified LPF-HA is prepared by subjecting an LPF-HA-containing solution to an affinity chromatography using a denatured ceruloplasmin as a ligand to adsorb the LPF-HA and eluting the LPF-HA.

15. The method according to claim 14, wherein the affinity chromatography is carried out on an LPF-HA-containing solution regulated to pH 4.0 to 10.0.

16. The method according to claim 14, wherein the denatured ceruloplasmin is a human- or other animal-origin ceruloplasmin.

17. The method according to claim 14, wherein the denatured ceruloplasmin is obtained by heat treatment of a human- or other animal-origin ceruloplasmin at 60 to 85 $^{\circ}$ C for 1 to 24 hours.

18. The method according to claim 14, wherein the denatured ceruloplasmin is obtained by treating a human- or other animal-origin ceruloplasmin with a reducing agent, a cyano compound, or a chelating agent.

19. The method according to claim 14, wherein the LPF-HA adsorbed on the ligand is eluted with a chaotropic base, an ethylene glycol, dioxane, urea, guanidine hydrochloride, or EDTA.

20. The method according to claim 1, wherein the purified LPF-HA is prepared by contacting an LPF-HA-containing solution with a cellulose sulfate gel, a polysaccharide gel chemically bound with dextran sulfate or a crosslinked polysaccharide sulfate gel to adsorb the LPF-HA and eluting the adsorbed LPF-HA from the gel.

21. The method according to claim 20, wherein the adsorption is carried out under the conditions of a pH of 5.0 to 5.0, a temperature of 0$^{\circ}$ to 30$^{\circ}$ C and a specific conductivity of 0.5 to 5.0 mS/cm.

22. The method according to claim 20, wherein the elution or LPF-HA from the gel is carried out with a buffer having a specific conductivity of 5.0 to 100.0 mS/cm.

23. The method according to claim 22, wherein the LPF-HA-adsorbed gel is washed with a buffer having a specific conductivity of 0.5 to 5.0 mS/cm before the elution.

24. The method according to claim 20, wherein the cellulose sulfate is a sulfuric acid ester of a crystalline cellulose or a cellulose having crystalline area and non-crystalline area.

25. The method according to claim 20, wherein the polysaccharide gel chemically bound with dextran sulfate is a dextran sulfate-agarose gel, a dextran sulfate-dextran gel, or a dextran sulfate-cellulose gel.

26. The method according to claim 20, wherein the crosslinked polysaccharide sulfate is a crosslinked cellulose sulfate, a crosslinked agarose sulfate, or a crosslinked dextran sulfate.

33

27. The method according to claim 26, wherein the crosslinked cellulose sulfate is an epichlorohydrin-crosslinked cellulose sulfate.

28. The method according to claim 26, wherein the crosslinked agarose sulfate is an epichlorohydrin-crosslinked agarose sulfate.

29. The method according to claim 26, wherein the crosslinked dextran sulfate is an epichlorohydrin-crosslinked dextran sulfate.

30. The method according to claim 1, wherein the toxoid or F-HA or LPF-Ha is prepared by a conventional formalinization.

31. A method for preparing a combined vaccine of a pertussis antigen, diphtheria toxoid and tetanus toxoid, which comprises preparing a pertussis component vaccine by mixing a purified filamentous hemagglutinin (F-HA) or a toxoid thereof and a purified leucocytosis-promoting factor hemagglutinin (LPF-HA) toxoid which are obtained from a culture medium of Bordetella pertussis, said purified F-HA being prepared by contacting an F-HA-containing solution with a cellulose sulfate gel, a polysaccharide gel chemically bound with dextran sulfate or a crosslinked polysaccharide sulfate gel to adsorb the F-HA and eluting the adsorbed F-HA from the gel, and then adding a diphtheria toxoid and a tetanus toxoid to the pertussis component vaccine.

**Revendications**

1. Un procédé pour fabriquer un composant de vaccin contre la coqueluche, par mélange d'une hémagglutinine filamenteuse (F-HA) purifiée ou d'un toxoïde de celle-ci et d'un toxoïde d'hémagglutinine du facteur activant la leucocytose (LPF-HA) purifié, que l'on obtient à partir d'un milieu de culture de Bordetella pertussis, ladite F-HA purifiée étant préparée par mise en contact d'une solution contenant la F-HA avec un gel de sulfate de cellulose, un gel de polysaccharide chimiquement lié à du sulfate de dextrane ou un gel de sulfate de polysaccharide réticulé pour adsorber la F-HA et élution de la F-HA adsorbée du gel.

2. Le procédé selon la revendication 1, dans lequel le gel de sulfate de cellulose, le gel de polysaccharide chimiquement lié à du sulfate de dextrane ou le gel de sulfate de polysaccharide réticulé est préalablement équilibré par traitement avec un tampon ayant un pH de 6,0 à 9,0 et une conductivité spécifique de 5,0 à 25/0 mS/cm et ensuite soumis à l'adsorption de la F-HA.

3. Le procédé selon la revendication 1, dans lequel l'adsorption est mise en oeuvre à un pH de 6,0 à 8,0, à une température de 0 à 30°C et à une conductivité spécifique de 5,0 à 25,0 mS/cm.

4. Le procédé selon la revendication 1, dans lequel l'élution de la F-HA du gel est mise en oeuvre avec un tampon ayant un pH de 5,0 et 10,0 et une conductivité spécifique de 25,0 à 130 mS/cm.

5. Le procédé selon la revendication 4, dans lequel le gel ayant adsorbé la F-HA est lavé par un tampon ayant un pH de 5,0 à 10,0 et une conductivité spécifique de 5,0 à 25,0 mS/cm avant l'élution.

6. Le procédé selon la revendication 1, dans lequel le sulfate de cellulose est un ester d'acide sulfurique d'une cellulose cristalline ou d'une cellulose ayant une zone cristalline et une zone non cristalline.

7. Le procédé selon la revendication 1, dans lequel le gel de polysaccharide chimiquement lié au sulfate de dextrane est un gel de sulfate de dextrane-agarose, un gel de sulfate de dextrane-dextrane ou un gel de sulfate de dextrane-cellulose.

8. Le procédé selon la revendication 1, dans lequel le sulfate de polysaccharide réticulé est un sulfate de dextrane réticulé, un sulfate d'agarose réticulé ou un sulfate de cellulose réticulé.

9. Le procédé selon la revendication 8, dans lequel le sulfate de dextrane réticulé est un sulfate de dextrane réticulé par l'épichlorhydrine.

10. Le procédé selon la revendication 8, dans lequel le sulfate d'agarose réticulé est un sulfate de d'agarose réticulé par l'épichlorhydrine.

11. Le procédé selon la revendication 8, dans lequel le sulfate de cellulose réticulé est un sulfate de celluose réticulé par l'épichlorhydrine.

12. Le procédé selon la revendication 1, dans lequel la LPF-HA purifiée est préparée en soumettant une solution contenant la LPF-HA à une chromatographie d'affinité utilisant l'haptoglobine ou la céruloplasmine comme ligand pour adsorber la LPF-HA et en éluant la LPF-HA.

13. Le procédé selon la revendication 12, dans lequel la solution contenant la LPF-HA est partiellement purifiée par traitement par une hydroxyapatite avant la chromatographie d'affinité utilisant comme ligand l'haptoglobine ou la céruloplasmine.

14. Le procédé selon la revendication 1, dans lequel la LPF-HA purifiée est préparée en soumettant une solution contenant la LPF-HA à une chromatographie d'affinité utilisant comme ligand une céruloplasmine dénaturée pour adsorber la LPF-HA et en éluant la LPF-HA.

15. Le procédé selon la revendication 14, dans lequel la chromatographie d'affinité est mise en oeuvre sur une solution contenant la LPF-HA réglée à pH 4,0-10,0.

16. Le procédé selon la revendication 14, dans lequel la céruloplasmine dénaturée est une céruloplasmine d'origine humaine ou d'un autre animal.

17. Le procédé selon la revendication 14, dans lequel la céruloplasmine dénaturée est obtenue par traitement thermique d'une céruloplasmine d'origine humaine ou animale à 60-85° C pendant 1 à 24 h.

18. Le procédé selon la revendication 14, dans lequel la céruloplasmine dénaturée est obtenue par traitement d'une céruloplasmine d'origine humaine ou animale par un agent réducteur, un composé cyano ou un agent chélatant.

19. Le procédé selon la revendication 14, dans lequel la LPF-HA adsorbée sur le ligand est éluée par une base chaotropique, un éthylèneglycol, le dioxanne, l'urée, le chlorhydrate de guanidine ou l'EDTA.

20. Le procédé selon la revendication 1, dans lequel la LPF-HA purifiée est préparée par mise en contact d'une solution contenant la LPF-HA avec un gel de sulfate de cellulose, un gel de polysaccharide chimiquement lié au sulfate de dextrane ou un gel de sulfate de polysaccharide réticulé pour adsorber la LPF-HA et en éluant la LPF-HA adsorbée du gel.

21. Le procédé selon la revendication 20, dans lequel l'adsorption est mise en oeuvre à un pH de 5,0 à 9,0, à une température de 0 à 30° C et à une conductivité spécifique de 0,5 à 5,0 mS/cm.

22. Le procédé selon la revendication 20, dans lequel l'élution de la LPF-HA du gel est mise en oeuvre avec un tampon ayant une conductivité spécifique de 5,0 à 100,0 mS/cm.

23. Le procédé selon la revendication 22, dans lequel le gel ayant adsorbé la LPF-HA est lavé avec un tampon ayant une conductivité spécifique de 0,5 à 5,0 mS/cm avant l'élution.

24. Le procédé selon la revendication 20, dans lequel le sulfate de cellulose est un ester d'acide sulfurique d'une cellulose cristalline ou d'une cellulose ayant une zone cristalline et une zone non cristalline.

25. Le procédé selon la revendication 20, dans lequel le gel de polysaccharide chimiquement lié au sulfate de dextrane est un gel de sulfate de dextrane-agarose, un gel d'un sulfate de dextrane -dextrane ou un gel de sulfate de dextrane -cellulose.

26. Le procédé selon la revendication 20, dans lequel le sulfate de polysaccharide réticulé est un sulfate de cellulose réticulé, un sulfate d'agarose réticulé ou un sulfate de dextrane réticulé.

35

27. Le procédé selon la revendication 26, dans lequel le sulfate de cellulose réticulé est un sulfate de cellulose réticulé par l'épichlorhydrine

28. Le procédé selon la revendication 26, dans lequel le sulfate d'agarose réticulé est un sulfate d'agarose réticulé par l'épichlorhydrine.

29. Le procédé selon la revendication 26, dans lequel le sulfate de dextrane réticulé est un sulfate de dextrane réticulé par l'épichlorhydrine.

30. Le procédé selon la revendication 1, dans lequel le toxoïde de la F-HA ou de la LPF-HA est préparé par une formalisation classique.

31. Un procédé pour fabriquer un vaccin combiné d'un antigène de la coqueluche, d'un toxoïde de la diphtérie et d'un toxoïde du tétanos, qui comprend la préparation d'un composant de vaccin contre la coqueluche par mélange d'une hémagglutinine filamenteuse (F-HA) purifiée ou d'un toxoïde de celle-ci et d'un toxoïde d'hémagglutinine du facteur activant la leucocytose (LPF-HA) purifié que l'on obtient à partir d'un milieu de culture de Bordetella pertussis, ladite F-HA purifiée étant préparée par mise en contact d'une solution contenant la F-HA avec un gel de sulfate de cellulose, un gel de polysaccharide chimiquement lié à du sulfate de dextrane ou un gel de sulfate de polysaccharide réticulé pour adsorber la F-HA et élution de la F-HA adsorbée du gel et ensuite addition d'un toxoïde de la diphtérie et d'un toxoïde du tétanos au composant de vaccin contre la coqueluche.

## Ansprüche

1. Verfahren zur Herstellung eines Pertussis-Komponenten-Impfstoffes, das das Mischen eines gereinigten filamentösen Hemagglutinins (F-HA), oder eines Toxoids davon und eines gereinigten Leucocytose-fördernden Faktor Hemagglutinin (LPF-HA)-Toxoids, die aus einem Kulturmedium von Bordetella pertussis erhalten werden, umfaßt, wobei das gereinigte F-HA durch Zusammenbringen einer F-HA enthaltenden Lösung mit einem Cellulosesulfatgel, einem an Dextransulfat chemisch gebundenen Polysaccharidgel oder einem vernetzten Polysaccharidsulfatgel zur Adsorption des F-HA, und Elution des adsorbierten F-HA aus dem Gel hergestellt wird.

2. Verfahren nach Anspruch 1, wobei das Cellulosesulfatgel, das an Dextransulfat chemisch gebundene Polysaccharidgel oder das vernetzte Polysaccharidsulfatgel zuerst durch Behandlung mit einem Puffer, der einen pH von 6,0 bis 9,0 und eine spezifische Leitfähigkeit von 5,0 bis 25,0 mS/cm aufweist, äquilibriert wird und dann der Adsorption von F-HA unterworfen wird.

3. Verfahren nach Anspruch 1, wobei die Adsorption bei einem pH von 6,0 bis 8,0, einer Temperatur von 0° bis 30° C und einer spezifischen Leitfähigkeit von 5,0 bis 25,0 mS/cm durchgeführt wird.

4. Verfahren nach Anspruch 1, wobei die Elution von F-HA aus dem Gel mit einem Puffer, der einen pH von 5,0 bis 10,0 und eine spezifische Leitfähigkeit von 25,0 bis 130 mS/cm aufweist, durchgeführt wird.

5. Verfahren nach Anspruch 4, wobei das F-HA-adsorbierte Gel mit einem Puffer, der einen pH von 5,0 bis 10,0 und eine spezifische Leitfähigkeit von 5,0 bis 25,0 mS/cm aufweist, vor der Elution gewaschen wird.

6. Verfahren nach Anspruch 1, wobei das Cellulosesulfat ein Schwefelsäureester einer kristallinen Cellulose oder einer Cellulose mit kristallinen und nicht-kristallinen Bereichen ist.

7. Verfahren nach Anspruch 1, wobei das an Dextransulfat chemisch gebundene Polysaccharidgel ein Dextransulfat-Agarosegel, ein Dextransulfat-Dextrangel oder ein Dextransulfat-Cellulosegel ist.

8. Verfahren nach Anspruch 1, wobei das vernetzte Polysaccharidsulfat ein vernetztes Dextransulfat, ein vernetztes Agarosesulfat oder ein vernetztes Cellulosesulfat ist.

9. Verfahren nach Anspruch 8, wobei das vernetzte Dextransulfat ein Epichlorhydrin-vernetztes Dextransulfat ist.

10. Verfahren nach Anspruch 8, wobei das vernetzte Agarosesulfat ein Epichlorhydrin-vernetztes Agarosesulfat ist.

11. Verfahren nach Anspruch 8, wobei das vernetzte Cellulosesulfat ein Epichlorhydrin-vernetztes Cellulosesulfat ist.

12. Verfahren nach Anspruch 1, wobei das gereinigte LPF-HA durch eine Affinitätschromatographie einer LPF-HA-enthaltenden Lösung, die Haptoglobin oder Ceruloplasmin als Ligand verwendet, um das LPF-HA zu adsorbieren, und durch Elution des LPF-HA hergestellt wird.

13. Verfahren nach Anspruch 12, wobei die LPF-HA-enthaltende Lösung teilweise durch Behandlung mit Hydroxyapatit vor der Affinitätschromatographie, die Haptoglobulin oder Ceruloplasmin als Ligand verwendet, gereinigt wird.

14. Verfahren nach Anspruch 1, wobei das gereinigte LPF-HA durch eine Affinitätschromatographie einer LPF-HA-enthaltenden Lösung, die denaturiertes Ceruloplasmin als Ligand verwendet, um das LPF-HA zu adsorbieren, und durch Elution des LPF-HA hergestellt wird.

15. Verfahren nach Anspruch 14, wobei die Affinitätschromatographie mit einer LPF-HA-enthaltenden Lösung, die auf pH 4,0 bis 10,0 eingestellt ist, durchgeführt wird.

16. Verfahren nach Anspruch 14, wobei das denaturierte Ceruloplasmin ein menschliches oder anderes tierisches Ceruloplasmin ist.

17. Verfahren nach Anspruch 14, wobei das denaturierte Ceruloplasmin durch Hitzebehandlung eines menschlichen oder anderen tierischen Ceruloplasmins bei 60° bis 85°C für 1 bis 24 Stunden erhalten wird.

18. Verfahren nach Anspruch 14, wobei das denaturierte Ceruloplasmin durch Behandlung eines menschlichen oder eines anderen tierischen Ceruloplasmins mit einem Reduktionsmittel, einer Cyano-Verbindung oder einem chelatbildenden Wirkstoff erhalten wird.

19. Verfahren nach Anspruch 14, wobei das an den Liganden adsorbierte LPF-HA mit einer chaotropen Base, Ethylenglykol, Dioxan, Harnstoff, Guadiniumhydrochlorid oder EDTA eluiert wird.

20. Verfahren nach Anspruch 1, wobei das gereinigte LPF-HA durch Zusammenbringen einer LPF-HA-enthaltenden Lösung mit einem Cellulosesulfatgel, einem an Dextransulfat chemisch gebundenen Polysaccharidgel oder einem vernetzten Polysaccharidsulfatgel, um das LPF-HA zu adsorbieren, und durch Elution des adsorbierten LPF-HA aus dem Gel hergestellt wird.

21. Verfahren nach Anspruch 20, wobei die Adsorption bei einem pH von 5,0 bis 9,0, einer Temperatur von 0° bis 30°C und einer spezifischen Leitfähigkeit von 0,5 bis 5,0 mS/cm durchgeführt wird.

22. Verfahren nach Anspruch 20, wobei die Elution von LPF-HA aus dem Gel mit einem Puffer, der eine spezifische Leitfähigkeit von 5,0 bis 100,0 mS/cm aufweist, durchgeführt wird.

23. Verfahren nach Anspruch 22, wobei das LPF-HA-adsorbierte Gel vor der Elution mit einem Puffer, der eine spezifische Leitfähigkeit von 0,5 bis 5,0 mS/cm aufweist, gewaschen wird.

24. Verfahren nach Anspruch 20, wobei das Cellulosesulfat ein Schwefelsäureester einer kristallinen Cellulose oder einer Cellulose mit kristallinen und nicht-kristallinen Bereichen ist.

25. Verfahren nach Anspruch 20, wobei das an Dextransulfat chemisch gebundene Polysaccharidgel ein Dextransulfat-Agarosegel, ein Dextransulfat-Dextrangel oder ein Dextransulfat-Cellulosegel ist.

26. Verfahren nach Anspruch 20, wobei das vernetzte Polysaccharidsulfat ein vernetztes Cellulosesulfat, ein

EP 0 175 841 B1

ver netztes Agarosesulfat oder ein vernetztes Dextransulfat ist.

27. Verfahren nach Anspruch 26, wobei das vernetzte Cellulosesulfat ein Epichlorhydrin-vernetztes Cellulosesulfat ist.

28. Verfahren nach Anspruch 26, wobei das vernetzte Agarosesulfat ein Epichlorhydrin-vernetztes Agarosesulfat ist.

29. Verfahren nach Anspruch 26, wobei das vernetzte Dextransulfat ein Epichlorhydrin-vernetztes Dextransulfat ist.

30. Verfahren nach Anspruch 1, wobei das Toxoid, F-HA oder LPF-HA durch eine übliche Formalinbehandlung hergestellt wird.

31. Verfahren zur Herstellung eines kombinierten Impfstoffes eines Pertussisantigens, Diphtherietoxoids und Tetanustoxoids, das die Herstellung eines Pertussis-Komponenten-Impfstoffes durch Mischen eines gereinigten filamentösen Hemagglutinins (F-HA), oder eines Toxoids davon und eines gereinigten Leukozytose fördernden Faktor Hemmagglutinin (LPF-HA)-Toxoids, die aus einem Kulturmedium von Bordetella pertussis erhalten werden, wobei das gereinigte F-HA durch Zusammenbringen einer F-HA enthaltenden Lösung mit einem Cellulosesulfatgel, einem an Dextransulfat chemisch gebundenen Polysaccharidgel oder einem vernetztem Polysaccharidsulfatgel zur Adsorption des F-HA, und Elution des adsorbierten F-HA aus dem Gel hergestellt wird, und dann die Zugabe eines Diphtherieund eines Tetanustoxoids zu dem Pertussis-Komponenten-Impfstoff umfaßt.

Fig. 1